Europäisches Patentamt

European Patent Office

Office européen des brevets

(11) Publication number: **0 177 942 B1**

# EUROPEAN PATENT SPECIFICATION

(45) Date of publication of patent specification: **01.04.92**  (51) Int. Cl.⁵: **A61B 8/06**

(21) Application number: **85112767.0**

(22) Date of filing: **08.10.85**

(54) Correlation detection type ultrasound blood flowmeter.

(30) Priority: **08.10.84 JP 210800/84**

(43) Date of publication of application:
**16.04.86 Bulletin 86/16**

(45) Publication of the grant of the patent:
**01.04.92 Bulletin 92/14**

(84) Designated Contracting States:
**DE FR GB NL SE**

(56) References cited:
**DE-B- 1 798 104       US-A- 3 762 221**
**US-A- 4 217 909       US-A- 4 324 258**
**US-A- 4 334 543       US-A- 4 417 584**

(73) Proprietor: **FUJITSU LIMITED**
**1015, Kamikodanaka Nakahara-ku**
**Kawasaki-shi Kanagawa 211(JP)**

(72) Inventor: **Amemiya, Shinichi c/o FUJITSU**
**LIMITED**
**Patent Department 1015 Kamikodanaka**
**Nakahara-ku Kawasaki-shi Kanagawa 211(JP)**

(74) Representative: **Sunderland, James Harry et al**
**HASELTINE LAKE & CO Hazlitt House 28**
**Southampton Buildings Chancery Lane**
**London WC2A 1AT(GB)**

EP 0 177 942 B1

**Description**

The present invention relates to an ultrasound blood flowmeter of a correlation detection type.

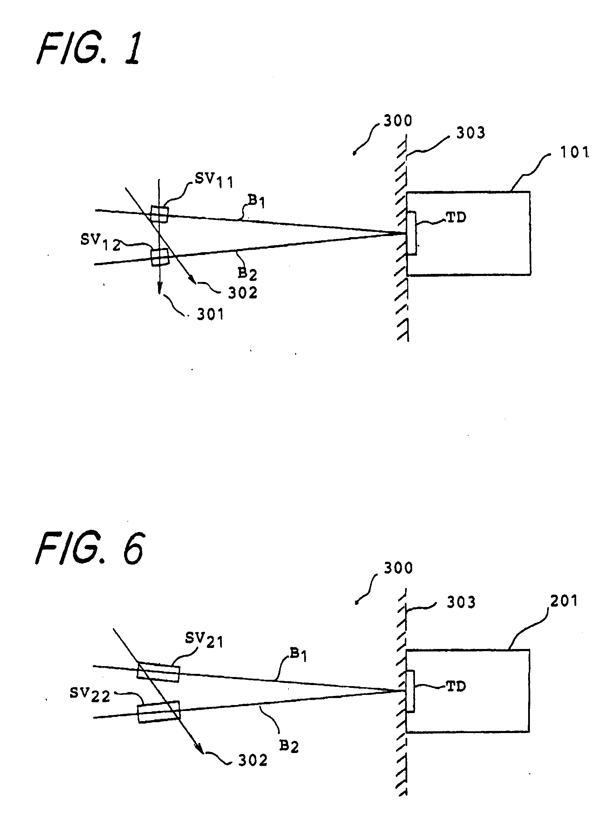

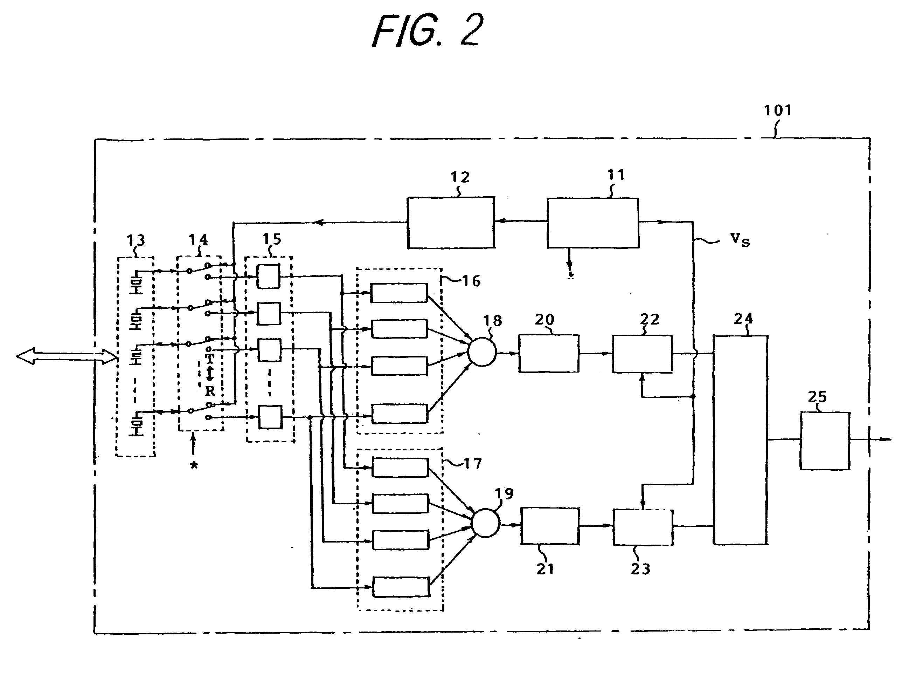

Usually, a Doppler effect type blood flowmeter is used in the measurement of blood flow speed. However, the speed measured by such a flowmeter is merely the component of blood flow velocity in the direction of a sound beam used in the measurement. In other words, speed (velocity component) perpendicular to the sound-beam cannot be measured by a Doppler effect type blood flowmeter. A correlation detection type blood flowmeter can measure the speed of a blood flow perpendicular to a measurement sound-beam. The correlation detection type blood flowmeter was invented by the inventor of the present invention and described in Japanese laid-open patent No. 58-71464 in 1983. Before discussing the present invention, that previously proposed correlation detection type blood flowmeter will be explained with reference to Figs. 1 and 2, to facilitate understanding of the present invention.

Fig. 1 is a schematic diagram illustrating the relationship between sound-beams ($B_1$ and $B_2$) formed by the previously proposed correlation detection type blood flowmeter 101 (blood flowmeter 101) and a blood flow 301 or 302 located in a human body 300.

The blood flowmeter 101 comprises an ultrasound transducer (transducer) TD placed on a surface 303 of the body 300; transducer TD forms dual sound-beams $B_1$ and $B_2$ for measuring the speed of blood flow 301, which flows almost perpendicularly with respect to sound-beams $B_1$ and $B_2$; transducer TD sends pulsed or burst ultrasound waves (burst sound waves) into human body 300 along sound-beams $B_1$ and $B_2$ and receives echo signals arising from reflections of the sound-beams by substances located along sound-beams $B_1$ and $B_2$.

$SV_{11}$ and $SV_{12}$ are called sample volumes and they relate to sound-beams $B_1$ and $B_2$ respectively. Sample volumes $SV_{11}$ and $SV_{12}$ are equally positioned (equally distant) from transducer TD. The location of the flowing blood is determined by other ultrasound imaging means, and the positions of sample volumes $SV_{11}$ and $SV_{12}$ are adjusted so that blood flow 301 is caught by (lies within) those sample volumes $SV_{11}$ and $SV_{12}$.

Fig. 2 is a schematic block diagram of blood flowmeter 101. In the Figure, 13 is an ultrasound transducer which corresponds to transducer TD in Fig. 1. Transducer 13 is a multi array type ultrasound transducer which consists of a plurality of transducer elements and operates rather like the familiar phased array antenna of a radar system.

Applying transducer 13 to blood flowmeter 101, blood flowmeter 101 may operate in two modes: a scanning mode and a focusing mode. The former mode is used for providing ultrasound imagery, such as B-mode imagery, and the latter is used for measuring blood speed. A change from one mode to the other can be easily effected by electronic means. Fig. 2 relates to the focusing mode in which two focused sound-beams $B_1$ and $B_2$ are formed. Actually, a blood flowmeter operates in combination with a unit for providing the scanning mode, but that is omitted in Fig. 2.

A control unit 11 generates timing and sampling control signals. The timing signal is for controlling transducer 13 and a switching circuit 14, and the sampling control signal is for controlling sample-and-hold (S-H) circuits 22 and 23. Under the control of control unit 11, a drive unit 12 outputs driving pulses for driving transducer 13 to send burst sound waves out from the transducer elements of transducer 13; the generated driving pulses synchronize with the timing signals from control unit 11. The switching unit 14 is for switching transducer 13 so as to send the burst sound waves and to receive reflected sound waves under the control of control unit 11. When switching unit 14 is turned to T (Fig. 2 shows this case) the transducer elements send the burst sound waves. After sending of the burst sound waves, switching unit 14 is turned to R, and then the transducer elements receive the reflected sound waves and convert them into electric echo signals (echo signals) respectively.

Echo signals transduced by transducer 13 are fed to amplifier elements of an amplifier 15 through switching unit 14 and fed to delay-line units 16 and 17. Delay-line units 16 and 17 correspond to sound-beams $B_1$ and $B_2$ respectively and each consists of delay-line elements which correspond to the transducer elements and compensate time differences of the received signals, so that the echo signals received along sound-beams $B_1$ and $B_2$ can be simply added by adders 18 and 19 respectively. Thus, the technique for timing the relation between the control time of respective driving pulse and the delay-time of respective echo signal provides sound-beams $B_1$ and $B_2$. The technique is similar to a phased array antenna technique for a radar system; the transducer elements are simultaneously driven, and burst sound waves are transmitted to (over) a rather broad area (range) covering both the sound-beams $B_1$ and $B_2$, but on the other hand respective receiving characteristics for the sound-beams $B_1$ and $B_2$ are made so as to be very sharp.

The echo signals added by adders 18 and 19 are fed to S-H circuits 22 and 23 through amplifiers 20

2

and 21 respectively. Each of the S-H circuits 22 and 23 is of a conventional type and echo signals respectively added by adders 18 and 19 are sampled at a sampling time $t_{sl}$ in accordance with a sampling control signal $V_S$ fed from control unit 11, and held. Sampling time $t_{sl}$ is determined by observing the location of the flowing blood, and sampling control signal $V_S$ is produced by manual adjustment of control unit 11. The determination of sampling time $t_{s1}$ is equivalent to the determination of the positions of sample volumes $SV_{11}$ and $SV_{12}$.

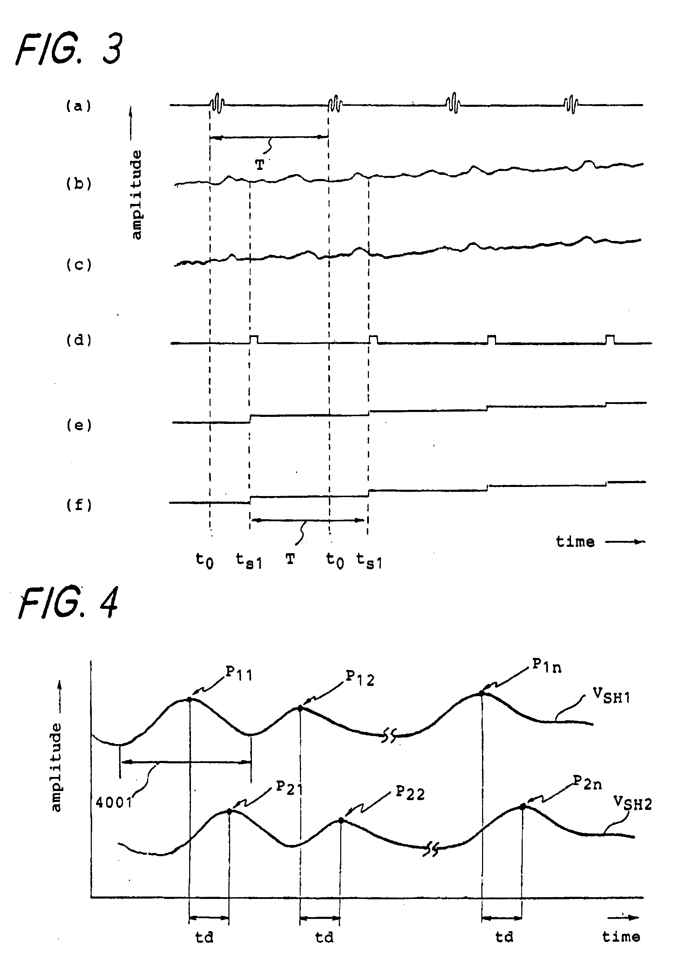

Fig. 3 is a waveform diagram illustrating mutual time relationships between burst sound waves, added echo signals, sampling control signals, and S-H voltages.

In Fig. 3, (a) is a train of burst sound waves, each of which bursts commences at a time $t_0$, having a period T; (b) and (c) show added echo signals with respect to sound-beams $B_1$ and $B_2$; (d) shows a train of sampling control signals, each being generated at time $t_{s1}$ counted from a corresponding time $t_0$; and (e) and (f) show S-H voltages $V_{SH1}$ and $V_{SH2}$ which correspond to sample volumes $SV_{11}$ and $SV_{12}$ respectively.

The S-H voltages $V_{SH1}$ and $V_{SH2}$ are fed to a time-difference detection circuit 24 which is for detecting a time difference between respective peak amplitudes of S-H voltages $V_{SH1}$ and $V_{SH2}$ by a cross correlation technique.

Fig. 4 illustrates a cross correlation relationship between peak amplitudes. As shown in Fig. 4, the amplitudes of S-H voltages $V_{SH1}$ and $V_{SH2}$ both have peaks. This is due to the fact that blood has a nature such that it flows in a state with small gathered-together masses of red blood corpuscles; the amplitude variation depends on the sizes of such masses. When blood flows almost perpendicularly with respect to sound-beams $B_1$ and $B_2$ passing through sample volumes $SV_{11}$ and $SV_{12}$, time-difference detection circuit 24 picks up peak amplitudes $P_{11}$ and $P_{21}$ from S-H voltages $V_{SH1}$ and $V_{SH2}$ respectively and detects a time-difference voltage $V_{td}$ between individual peak amplitudes $P_{11}$ (or $P_{12}$, --, or $P_{1n}$) and $P_{21}$ (or $P_{22}$, --, or $P_{2n}$) producing an output voltage called a time-difference voltage $V_{td}$. The time-difference direction circuit 24 comprises a fixed delay-line, a variable delay-line, and an automatic signal coincidence circuit; the details of which were given in Japanese laid-open patent No. 58-71464 in 1983. The time difference voltage $V_{td}$ is fed to a speed calculating circuit 25 in which the speed of blood which flows almost perpendicularly with respect to sound-beams $B_1$ and $B_2$ passing through sample volumes $SV_{11}$ and $SV_{12}$ is calculated. The calculation is performed by dividing a distance between sample volumes $SV_{11}$ and $SV_{12}$ by time difference $t_{td}$; the distance can be obtained in advance by determining the product of distance between transducer 13 and sample volume $SV_{11}$ or $SV_{12}$ and angle (radians) between sound-beams $B_1$ and $B_2$.

Thus, the correlation detection type blood flowmeter can measure the speed of blood which flows almost perpendicularly with respect to a measuring sound-beam. This is great advantage compared with the Doppler type blood flowmeter which can only measure the speed of blood flowing along a measuring sound-beam. However, in the previously proposed correlation detection type blood flowmeter, the sample volume $SV_{11}$ or $SV_{12}$ has almost no range to pick up the blood mass along the sound-beam, so that it has been very hard to detect the flowing blood, because it is hard to adjust sample volumes $SV_{11}$ and $SV_{12}$ so as to catch the flowing blood; there is often a case that the blood flows aslant to the sound-beam as shown by blood flow 302 in Fig. 1. This has been a problem with the previously proposed correlation detection type blood flowmeter. Furthermore, signal reflections caused by a fixed substance or body such as a blood vessel disturb detection of echo signals reflected by flowing blood, and this has been another problem of the previously proposed correlation detection type blood flowmeter.

The latter problem, of disturbance of detection of echo signals, is also known for instance in the field of Doppler blood flow meters. Solutions have been proposed involving selection of echo signals reflected by flowing blood, for example as described in US-A-4 334 534.

According to the present invention there is provided a correlation detection-type ultrasound blood flowmeter for measuring a component of speed of flowing blood, which component is perpendicular with respect to a centre line between two sound-beams received by said blood flowmeter, said flowmeter comprising a transducer for sending repeated bursts of ultrasound waves towards said flowing blood and receiving echo signals from said flowing blood when said flowing blood passes across two sample volumes which are equally distant from the transducer along respective ones of the two sound-beams, said speed of flowing blood being measured by cross correlation time-difference detection performed by using sample and hold voltages obtained in dependence upon said echo signals from said flowing blood passing across said two sample volumes, said blood flowmeter comprising:-

first means, for selecting from echo signals arising from reflections by any body or substance, including flowing blood and fixed bodies or substances, located along said two sound-beams only those echo signals arising from reflections by flowing blood which passes across said two sound-beams, by eliminating echo signals arising from reflections by fixed bodies or substances located along said two sound-beams; and

range gate means, for extending the range of each of said sample volumes in the direction of each respective sound beam to provide two extended sample volumes to sample and hold echo signals reflected by flowing blood which passes across said two extended sample volumes not only perpendicularly but also aslant with respect to said centre line between the directions of said two sound-beams.

An embodiment of the present invention can afford an improvement in relation to a correlation detection type blood flowmeter so that the blood flowmeter can measure the speed of flowing blood which flows not only perpendicularly with respect to but also aslant with respect to a sound-beam provided by the blood flowmeter.

An embodiment of the present invention can afford an improvement in relation to a blood flowmeter so that the blood flowmeter can be used to measure the speed of flowing blood with high accuracy and reliability by decreasing disturbances due to echo signals reflected by fixed bodies or substances located along the sound-beam.

In an embodiment of the present invention a range gate circuit and a quadrature detection circuit are employed in the blood flowmeter; the former is for increasing the serviceable range of a sample volume along a sound-beam, and the latter is for decreasing the disturbances due to echo signals reflected by fixed bodies or substances.

Reference is made, by way of example, to the accompanying drawings, in which:

Fig. 1 is a schematic diagram illustrating interrelationships between sample volumes, provided by the previously proposed correlation detection type blood flowmeter, and a blood flow;

Fig. 2 is a schematic block diagram of the previously proposed correlation detection type blood flowmeter;

Fig. 3 is a waveform diagram for assistance in illustrating operations of the previously proposed correlation detection type blood flowmeter, in which Figure

(a) is a waveform of burst sound waves,

(b) is a waveform of an echo signal with respect to a sound-beam $B_1$,

(c) is a waveform of an echo signal with respect to a sound-beam $B_2$,

(d) is a waveform of a train of sampling control signals,

(e) is a waveform of an S-H voltage with respect to sound-beam $B_1$, and

(f) is a waveform of an S-H voltage with respect to sound-beam $B_2$;

Fig. 4 is a waveform chart in a graphical form for illustrating a cross correlation between two S-H voltages;

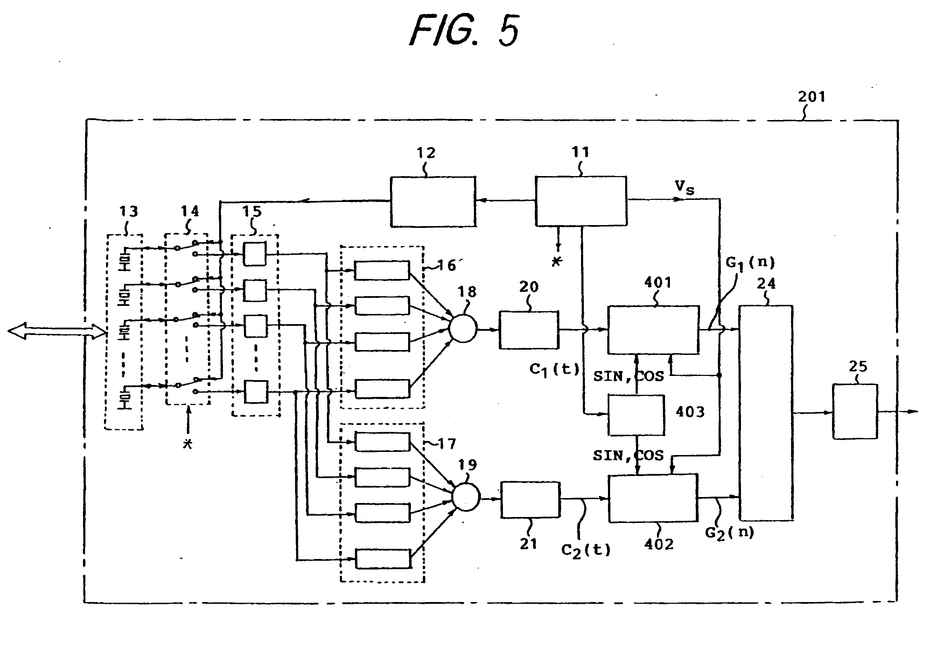

Fig. 5 is a schematic block diagram illustrating a correlation detection type blood flowmeter embodying the present invention;

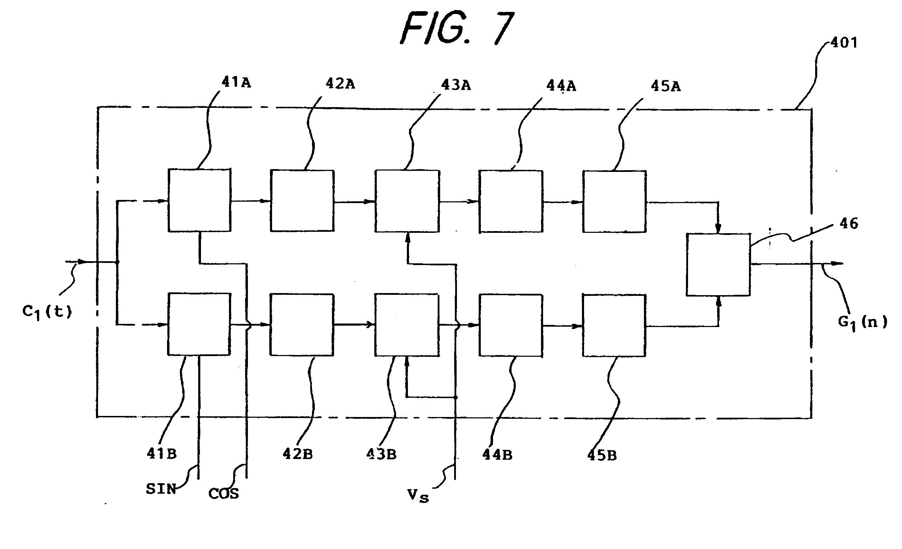

Fig. 6 is a schematic diagram illustrating interrelationships between sample volumes, provided by a correlation detection type blood flowmeter embodying the present invention, and a blood flow;

Fig. 7 is a schematic block diagram of a detection and gate unit of a correlation detection type blood flowmeter embodying the present invention;

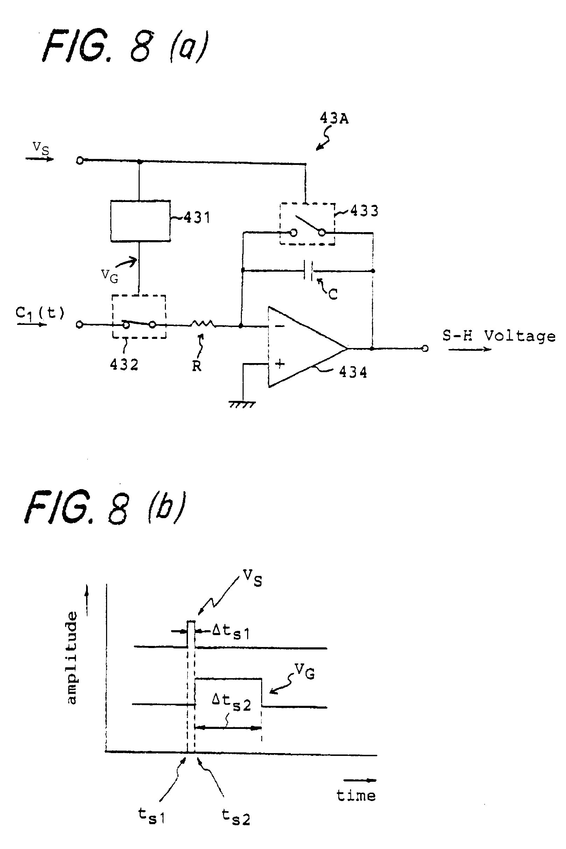

Fig. 8(a) is a schematic circuit diagram of a range gate circuit of a detection and gate unit of a correlation detection type blood flowmeter embodying the present invention; and

Fig 8(b) is a waveform chart in graphical form, illustrating operations of a range gate circuit of a correlation detection type blood flowmeter embodying the present invention.

In Fig. 5, which illustrates a correlation detection type blood flowmeter 201 (blood flowmeter 201) embodying the present invention, each block with the same reference sign as a block in Fig. 2 has the same or similar function to the block of Fig. 2. The blood flowmeter 201 comprises two quadrature-detection and range-gate units (detection and gate units) 401 and 402 and a sine-cosine signal generator (sin-cos generator) 403. The detection and gate units 401 and 402 and sine-cosine generator 403 are for providing improvement in relation to S-H circuits 22 and 23 of the previously proposed blood flowmeter 101 shown in Fig. 2. The detection and gate units 401 and 402 each have the same function; the former being for (performing the function in respect of) echo signals $C_1(t)$ received along sound-beam $B_1$ (see Fig. 6) and the latter being for echo signals $C_2(t)$ received along sound-beam $B_2$ (see Fig. 6).

Fig. 7 is a block diagram of detection and gate unit 401 in which echo signals reflected by fixed bodies or substances located along sound-beam $B_1$ are eliminated by a quadrature detection technique, and an echo signal reflected by flowing blood passing across sound-beam $B_1$ is gated over a relatively long range along sound-beam $B_1$ and sampled and held by a gated sample-and-holding technique. The long range corresponds to a sample volume $SV_{21}$ as shown in Fig. 6. Similarly, as seen in Fig. 6, a sample volume $SV_{22}$ along sound-beam $B_2$ can also have a long range. Thus, the ranges of sample volumes $SV_{21}$ and $SV_{22}$ can be extended respectively and accordingly the speed of blood can be measured even when the blood flows aslant with respect to sound-beams $B_1$ and $B_2$ as shown in Fig. 6.

In Fig. 7, detection and gate unit 401 comprises two multipliers 41A and 41B, two low-pass filters 42A

4

and 42B, two range gate circuits 43A and 43B, two high-pass filters 44A and 44B, and two square circuits 45A and 45B; and an adding circuit 46. In the above circuits, range gate circuit 43A (43B) and high-pass filter 44A (44B) are for gated sample-and-holding, and others are for quadrature detection.

When burst ultrasound waves are sent into a body, an echo signal reflected by a fixed body or substance such as a blood vessel is given by

$$A(t).\sin(\omega t + 0), \qquad (1)$$

and an echo signal reflected from flowing blood is given by

$$B(t + a_n).\sin(\omega t + b_n),$$

where,
t: time,
A: amplitude function of an echo signal reflected by a fixed body or substance,
B: amplitude function of an echo signal reflected by flowing blood,
$\omega$: angular velocity of ultrasound transmitted in the body,
a: a factor concerning the Doppler effect,
b: a factor concerning flowing blood velocity,
n: respective number of sequential driving pulses, and
0: a phase difference concerned with ultrasound as reflected by the fixed body or substance.

When echo signals presented by formulas (1) and (2) are superimposed (actual echo signals being superimposed) the following echo signal C(t) is obtained by adding formulas (1) and (2):

$$C(t) = A(t).\sin(\omega t + 0) + B(t + a_n).\sin(\omega t + b_n). \qquad (3)$$

In formula (3), a necessary term for measuring the blood speed is a term $B(t + a_n)$. However, as seen in formula (3) it is impossible to detect the term $B(t + a_n)$ simply from formula (3).

This can be solved by applying a quadrature detection technique as follows.

Firstly, multiply a sine signal and a cosine signal with echo signal C(t) so that following signals $D_1(t)$ and $D_2(t)$ are obtained:

$$
\begin{aligned}
D_1(t) &= C(t) \cdot \sin \omega t \\
&= A(t) \cdot \sin(\omega t + \theta) \cdot \sin \omega t \\
&\quad + B(t + a_n) \cdot \sin(\omega t + b_n) \cdot \sin \omega t \\
&= A(t)(-1/2 \cdot (\cos(2\omega t + \theta) - \cos \theta)) \\
&\quad + B(t - a_n)(-1/2 \cdot (\cos(2\omega t + b_n) - \cos b_n)), \qquad (4)
\end{aligned}
$$

and

$$
\begin{aligned}
D_2(t) &= C(t) \cdot \cos \omega t \\
&= A(t) \cdot \sin(\omega t + \theta) \cdot \cos \omega t \\
&\quad + B(t + a_n) \cdot \sin(\omega t + b_n) \cdot \cos \omega t \\
&= A(t) \cdot 1/2 \cdot (\sin(2\omega t + \theta) + \sin \theta) \\
&\quad + B(t + a_n) \cdot 1/2 \cdot (\sin(2\omega t + b_n) + \sin b_n). \qquad (5)
\end{aligned}
$$

Such sine and cosine signals are generated in sin-cos generator 403 in Fig. 5 and applied to detection and gate units 401 and 402 as shown in Fig. 5. The multiplications are effected by multipliers 41A and 41B respectively as shown in Fig. 7, and components of $\omega$ and $2\omega$ are removed by low-pass filters 42A and 42B.

Hence, the output signals from low-pass filters 42A and 42B respectively become

$$E_1(t) = 1/2 \cdot A(t) \cdot \cos \theta + 1/2 \cdot B(t + a_n) \cdot \cos b_n \qquad (6)$$

and

$$E_2(t) = 1/2 \cdot A(t) \cdot \sin \theta + 1/2 \cdot B(t + a_n) \cdot \sin b_n. \qquad (7)$$

Secondarily, the output signals $E_1(t)$ and $E_2(t)$ are fed to range gate circuits 43A and 43B respectively. The circuit structure and operation of range gate circuits 43A or 43B will be explained later but, to sum up, signals $E_1(t)$ and $E_2(t)$ are sampled from a series of incoming echo signals at $t_{s1}$ ($t = t_{s1}$) in every driving pulse interval (for every n) within a gated time interval which corresponds to $SV_{21}$, and held. (Fig. 7 relates to echo signal $C_1(t)$ - see Fig. 5 - and hence to sound-beam $B_1$ and $SV_{21}$). As explained with regard to Fig. 2, the sampling time $t_{s1}$ is determined by the distance between transducer 13 and the flowing blood, and sampling control signal $V_S$ for enabling sampling of echo signals at sampling time $t_{s1}$ is provided by control unit 11 and fed to detection and gate units 401 and 402 respectively as shown in Figs. 5 and 7. The output signals of range gate circuits 43A and 43B are fed to high-pass filters 44A and 44B as shown in Fig. 7, and the first terms of formulae (6) and (7) are removed to leave only the second terms. Hence, output signals $F_1$ and $F_2$ of high-pass filters 44A and 44B become respectively

$$F_1(n) = 1/2 \cdot B(T_{s1} + a_n) \cdot \cos b_n, \qquad (8)$$

and

$$F_2(n) = 1/2 \cdot B(t_{s1} + a_n) \cdot \sin b_n. \qquad (9)$$

The output signals $F_1$ and $F_2$ are fed to square circuits 45A and 45B respectively and added in adding circuit 36; hence, the following square sum is obtained:

$$G_1(n) = 1/4 \cdot B^2(t_{s1} + a_n). \qquad (10)$$

The square sum $G_1(n)$ consists only of term $B(t_{s1} + a_n)$, in other words, the required term $B(t_{s1} + a_n)$, which is concerned only with the flowing blood, can be obtained.

Fig. 8(a) is a schematic circuit diagram of range gate circuit 43A, which functionally consists of two circuits: a gated sampling circuit comprising a gate pulse generator 431, a switching element 432, and a switching element 433; and a holding circuit comprising an operational amplifier 434, a resistor R, and a capacitor C. Range gate circuit 43A operates as follows when it receives sampling control signal $V_S$ from control unit 11. Just before sampling control signal $V_S$ is applied to range gate circuit 43A, switching elements 432 and 433 are OFF, so that an output voltage (S-H voltage) of range gate circuit 43A is a voltage charged on capacitor C. When sampling control signal $V_S$, which is a pulse having a pulse width (period) $\Delta t_{s1}$ as shown in Fig. 8(b), is fed to range gate circuit 43A at time $t_{s1}$, switching element 432 is still OFF but switching element 433 becomes ON, so that the voltage charged on capacitor C is discharged; accordingly, the output of range gate circuit 43A achieves an initial potential. When sampling control signal $V_S$ is ended at time $t_{s2}$ as shown in Fig. 8(b), switching element 433 becomes OFF, and at the same time, gate pulse generator 431 generates a gate pulse $V_G$ having a pulse width (time duration) $\Delta t_{s2}$ and gate pulse $V_G$ is applied to switching element 432 so that switching element 432 becomes ON during time duration $\Delta t_{s2}$. Accordingly, mean value of the echo signals of sound-beam $B_1$ is held at time $t_{s2}$ on capacitor C during time duration $\Delta t_{s2}$. (A value determined by echo signals of sound-beam $B_1$ over the time duration $\Delta t_{s2}$ from an initial time $t_{s2}$ is thus stored in capacitor C). Time duration $\Delta t_{s2}$ is a sampling time duration which corresponds to the range of sample volume $SV_{21}$, and the voltage charged on capacitor C is the S-H voltage of range gate circuit 43A; the S-H voltage is fed to high-pass filter 44A as shown in Fig. 7.

The echo signal $C_2(t)$ is processed by detection and gate unit 402 similarly to the processing of $C1(t)$ by detection and gate unit 401, and a square sum $G_2(n)$ is obtained. Square sums $G_1(n)$ and $G_2(n)$ are fed to time-difference detection circuit 24 in which a time difference between square sum $G_1(n)$ and square sum $G_2(n)$ is detected. The output of time-difference detection circuit 24 is fed to a speed calculating circuit 25 in which the speed of blood which flows through sample volumes $SV_{21}$ and $SV_{22}$ is calculated. The operations of time-difference detection circuit 24 and speed calculation circuit 25 in blood flowmeter 201 are similar to such operations provided in the previously proposed blood flowmeter 101.

As will be clear from the above discussion, blood flowmeter 201 is able to measure speed in respect of blood which flows perpendicularly with respect to the sound-beams, or blood which flows aslant to the sound-beams. However, in this connection the word "speed" should be understood as "a component of speed (velocity) perpendicular to the sound-beam" to be exact, because when blood flows aslant to the sound-beam, the direction of the blood speed is also slant to the sound-beam; however, as seen in the above discussion, blood flowmeter 201 only measures "a component of speed perpendicular to the sound-beam".

The quadrature detection and correlation detection technique applied to blood flowmeter 201 can be used for measuring speed of blood which flows along a sound-beam. In this case, two sample volumes are properly positioned "along" the single sound-beam; in this case the sample volumes need not be so long as used in blood flowmeter 201; the simple sample and hold technique can be used instead of the range gate circuit used in blood flowmeter 201, as is employed in the previously proposed flowmeter 101.

Accordingly, true speed of blood which flows aslant with respect to a sound-beam can be obtained by calculating a vector sum of a horizontal speed component measured by blood flowmeter 201 and a vertical speed component measured using the quadrature detection and correlation detection technique, or measured by a Doppler type blood flowmeter.

## Claims

1.  A correlation detection-type ultrasound blood flowmeter (201) for measuring a component of speed of flowing blood (302), which component is perpendicular with respect to a centre line between two sound-beams (B1, B2) received by said blood flowmeter, said flowmeter comprising a transducer (TD) for sending repeated bursts of ultrasound waves towards said flowing blood and receiving echo signals from said flowing blood when said flowing blood passes across two sample volumes (SV21, SV22) which are equally distant from the transducer along respective ones of the two sound-beams, said speed of flowing blood being measured by cross correlation time-difference detection performed by using sample and hold voltages obtained in dependence upon said echo signals from said flowing blood passing across said two sample volumes, said blood flowmeter comprising:-

    first means (401, 402, 403), for selecting from echo signals arising from reflections by any body or substance, including flowing blood and fixed bodies or substances, located along said two sound-beams only those echo signals arising from reflections by flowing blood which passes across said two sound-beams, by eliminating echo signals arising from reflections by fixed bodies or substances located along said two sound-beams; and

    range gate means (401-43A, 43B; 402), for extending the range of each of said sample volumes in the direction of each respective sound beam to provide two extended sample volumes to sample and hold echo signals reflected by flowing blood which passes across said two extended sample volumes not only perpendicularly but also aslant with respect to said centre line between the directions of said two sound-beams.

2.  A correlation detection-type ultrasound blood flowmeter according to claim 1, wherein said first means comprises two quadrature detection circuits (401, 402) and a sine-cosine signal generator (403), said sine-cosine signal generator (403) being provided for applying a sine signal and a cosine signal to each of the two quadrature detection circuits (401, 402), and the two quadrature detection circuits being provided for respective ones of the said two sound-beams, each quadrature detection circuit (401, 402) comprising:-

    two multipliers (41A, 41B) for multiplying echo signals arising from reflections by any body or substance located along the relevant sound-beam by said sine signal and said cosine signal respectively;

    two low-pass filters (42A, 42B) for eliminating high frequency components included in the output signals of said two multipliers (41A, 41B) and providing the low frequency components to said range gate means (401-43A, 43B; 402), said high frequency components being concerned with reflections from all bodies or substances located along the relevant sound-beam;

    two high-pass filters (44A, 44B) for eliminating low frequency components of output signals of said range gate means (401-43A, 43B; 402), said low frequency components being concerned with reflections from fixed bodies or substances located along the relevant sound-beam;

    two square circuits (45A, 45B) for squaring output signals of said two high-pass filters (44A, 44B); and

an adding circuit (46) for adding output signals of said two square circuits (45A, 45B) to produce a signal voltage relating to said flowing blood located along the relevant sound-beam.

**3.** A correlation detection-type ultrasound blood flowmeter according to claim 2, wherein said range gate comprises four range gate circuits (401-43A, 43B; 402), two of said four range gate circuits being inserted in each quadrature detection circuit of the first means (401, 402), between said two low-pass filters (42A, 42B) and said two high-pass filters (44A, 44B) of the quadrature detection circuit concerned,

each said range gate circuit (43A, 43B) comprising a holding circuit (434, R, C) and a gated sampling circuit (431, 432, 433);

said holding circuit comprising an operational amplifier (434), a resistor (R) connected to an input terminal of said operational amplifier, and a capacitor (C) connected between said input terminal of said operational amplifier and an output terminal of said operational amplifier, said output terminal corresponding to an output terminal of said range gate circuit;

said gated sampling circuit comprising:-

a gate pulse generator (431) for generating a gate pulse, which has a first pulse width which corresponds to said extended sample volume, every time a sampling control pulse signal having a second pulse width is applied to said gate pulse generator for designating the position of said sample volume on the relevant sound-beam with a repetition frequency equal to that of said bursts of ultrasound waves and when said second pulse width is ended;

a first switching element (432) for making an input connection, for leading output signals of a low-pass filter (42A, 42B) of a quadrature detection circuit to said resistor (R) of said holding circuit, ON when said gate pulse is applied to said first switching element during a time of said first pulse width; and

a second switching element (433) for making a short connection, for discharging a voltage charged on said capacitor (C) of said holding circuit, ON when said sampling control signal is applied to said second switching element during a time of said second pulse width.

## Revendications

**1.** Un appareil de mesure du débit sanguin à ultrasons, du type à détection de corrélation (201), prévu pour la mesure d'une composante de vitesse du sang en circulation (302), cette composante étant perpendiculaire à un axe qui s'étend entre deux faisceaux sonores ($B_1$, $B_2$) qui sont reçus par l'appareil de mesure de débit sanguin, cet appareil de mesure de débit sanguin comprenant un transducteur (TD) qui est destiné à émettre des salves répétées d'ondes ultrasonores vers le sang en circulation et à recevoir des signaux d'écho provenant du sang en circulation, lorsque ce dernier traverse deux volumes d'échantillons ($SV_{21}$, $SV_{22}$) qui sont équidistants du transducteur le long de faisceaux respectifs parmi les deux faisceaux sonores, la vitesse du sang en circulation étant mesurée par une détection de différence de temps d'inter-corrélation qui est effectuée en employant des tensions échantillonnées et bloquées qui sont obtenues sous la dépendance des signaux d'écho provenant du sang en circulation qui traverse les deux volumes d'échantillons, cet appareil de mesure de débit sanguin comprenant :

des premiers moyens (401, 402, 403) destinés à sélectionner, parmi des signaux d'écho qui résultent de réflexions sur n'importe quel corps ou substance, comprenant le sang en circulation et des corps ou des substances fixes, se trouvant le long des deux faisceaux sonores, seulement les signaux d'écho qui résultent de réflexions par le sang en circulation qui traverse les deux faisceaux sonores, par l'élimination des signaux d'écho qui résultent de réflexions par des corps ou des substances fixes se trouvant le long des deux faisceaux sonores; et

des moyens de définition de fenêtre de distance (401-43A, 43B; 402), destinés à étendre la longueur de chacun des volumes d'échantillons dans la direction de chaque faisceau sonore respectif, pour donner deux volumes d'échantillons étendus, dans le but d'échantillonner et de bloquer des signaux d'écho qui sont réfléchis par le sang en circulation qui traverse les deux volumes d'échantillons étendus, non seulement perpendiculairement mais également obliquement par rapport à l'axe situé entre les directions des deux faisceaux sonores.

**2.** Un appareil de mesure de débit sanguin à ultrasons, du type à détection de corrélation, selon la revendication 1, dans lequel les premiers moyens comprennent deux circuits de détection en quadrature (401, 402) et un générateur de signal en sinus-cosinus (403), ce générateur de signal en sinus-cosinus (403) étant destiné à appliquer un signal en sinus et un signal en cosinus à chacun des deux circuits de détection en quadrature (402, 402), et les deux circuits de détection en quadrature étant établis pour des faisceaux respectifs parmi les deux faisceaux sonores, chaque circuit de détection en quadrature (401, 402) comprenant :

deux multiplieurs (41A, 41B) qui sont destinés à multiplier des signaux d'écho résultant de réflexions par un corps ou une substance quelconque se trouvant le long du faisceau sonore considéré, respectivement par le signal en sinus et le signal en cosinus;

deux filtres passe-bas (42A, 42B) destinés à éliminer des composantes de fréquence élevée qui sont incluses dans les signaux de sortie des deux multiplieurs (41A, 41B), et à fournir les composantes de fréquence basse aux moyens de définition de fenêtre de distance (401-43A, 43B; 402), ces composantes de fréquence élevée correspondant à des réflexions sur tous les corps ou substances qui se trouvent le long du faisceau sonore considéré;

deux filtres passe-haut (44A, 44B) qui sont destinés à éliminer les composantes de fréquence basse des signaux de sortie des moyens de définition de fenêtre de distance (401-43A, 43B; 402), ces composantes de fréquence basse correspondant à des réflexions sur des corps ou des substances fixes se trouvant le long du faisceau sonore considéré;

deux circuits d'élévation au carré (45A, 45B) qui sont destinés à élever au carré les signaux de sortie des deux filtres passe-haut (44A, 44B); et

un circuit additionneur (46) destiné à additionner les signaux de sortie des deux circuits d'élévation au carré (45A, 45B), pour produire une tension de signal relative au sang en circulation qui se trouve le long du faisceau sonore considéré.

3. Un appareil de mesure de débit sanguin à ultrasons, du type à détection de corrélation, selon la revendication 2, caractérisé en ce que les moyens de définition de fenêtre de distance comprennent quatre circuits de fenêtre de distance (401-43A, 43B; 402), deux de ces quatre circuits de fenêtre de distance étant incorporés dans chaque circuit de détection en quadrature des premiers moyens (401, 402), entre les deux filtres passe-bas (42A, 42B) et les deux filtres passe-haut (44A, 44B) du circuit de détection en quadrature considéré,

chaque circuit de fenêtre de distance (43A, 43B) comprenant un circuit bloqueur (434, R, C) et un circuit d'échantillonnage avec fenêtre (431, 432, 433);

le circuit bloqueur comprenant un amplificateur opérationnel (434), une résistance (R) connectée à une borne d'entrée de l'amplificateur opérationnel, et un condensateur (C) connecté entre la borne d'entrée de l'amplificateur opérationnel et une borne de sortie de l'amplificateur opérationnel, cette borne de sortie correspondant à une borne de sortie du circuit de fenêtre de distance;

le circuit d'échantillonnage avec fenêtre comprenant :

un générateur d'impulsions de fenêtre (431) destiné à générer une impulsion de fenêtre, qui a une première largeur d'impulsion qui correspond au volume d'échantillon étendu, chaque fois qu'un signal d'impulsion de commande d'échantillonnage ayant une seconde largeur d'impulsion est appliqué au générateur d'impulsions de fenêtre, pour désigner la position du volume d'échantillon sur le faisceau sonore considéré, avec une fréquence de répétition égale à celle des salves d'ondes ultrasonores, et lorsque la durée correspondant à la seconde largeur d'impulsion est terminée;

un premier élément de commutation (432) destiné à établir une connexion d'entrée, pour acheminer des signaux de sortie d'un filtre passe-bas (42A, 42B) d'un circuit de détection en quadrature vers la résistance (R) du circuit bloqueur, ce premier élément de commutation étant à l'état conducteur lorsque l'impulsion de fenêtre lui est appliquée pendant un intervalle de temps correspondant à la première largeur d'impulsion; et

un second élément de commutation (433) destiné à établir une connexion de court-circuit, pour décharger une tension de charge du condensateur (C) du circuit bloqueur, ce second élément de commutation étant à l'état conducteur lorsque le signal de commande d'échantillonnage lui est appliqué pendant un intervalle de temps correspondant à la seconde largeur d'impulsion.

**Patentansprüche**

1. Korrelationserkennungstyp-Ultraschall-Blutflußmesser (201) zur Messung einer Komponente des fließenden Blutes (302), welche Komponente senkrecht ist, bezogen auf die Mittellinie zwischen zwei Schallstrahlen (B1, B2), die das Blutflußmesser empfängt, welches Flußmesser einen Wandler (TD) umfaßt zum Senden von wiederholten Stößen von Ultraschallwellen zu dem fließenden Blut und zum Empfangen von Echosignalen von dem fließenden Blut, wenn das fließende Blut durch zwei Probevolumina (SV 21, SV 22) hindurchtritt, welche entlang jeweils einem der zwei Schallstrahlen gleichweit vom Wandler (TD) entfernt sind, welche Geschwindigkeit des fließenden Blutes mittels Kreuzkorrelations-Zeitdifferenzerkennung gemessen wird, die durchgeführt wird unter Verwendung der Abtast- und Haltespannungen, die in Abhängigkeit von den Echosignalen des durch die zwei Probevolumina hindurchtretenden, fließenden Blut erhalten wird, welches Blutflußmesser umfaßt :

erste Mittel (401, 402, 403) zum Auswählen aus Echosignalen, die von Reflexionen an irgendeinem Körper oder einer Substanz herrühren, einschließlich fließenden Blutes und festen Körper oder Substanzen, die entlang der zwei Schallstrahlen angeordnet sind, nur solcher Echosignale, die von Reflexionen am fließenden Blut, welches durch die zwei Schallstrahlen hindurchtritt, herrühren, durch Eliminieren von Echosignalen, die von Reflexionen an festen Körpern oder Substanzen, die entlang den Ultraschallstrahlen angeordnet sind, herrühren; und

Bereichsgattermittel (401-43A, 43B; 402), zum Ausdehnen des Bereiches jedes der Probevolumina in Richtung jedes jeweiligen Schallstrahls, um zwei ausgedehnten Probevolumina mit Abtast-und Halt-Echosignalen vorzusehen, die vom fließendem Blut reflektiert sind, welches durch die zwei ausgedehnten Probevolumina nicht nur senkrecht sondern auch schräg, bezogen auf die Mittellinie zwischen den Richtungen der zwei Schallstrahlen, hindurchtritt.

2. Korrelationserkennungstyp-Ultraschall-Blutflußmesser nach Anspruch 1, bei dem das erste Mittel zwei Quadraturerkennungsschaltungen (401, 402) und einen Sinus-Cosinus-Signalgenerator (403) enthält, der Sinus-Cosinus-Signalgenerator (403) ist zum Aufgeben eines Sinus-Signals und eines Cosinus-Signals auf jede der zwei Quadraturerkennungsschaltungen (401, 402) vorgesehen ist und die zwei Quadraturerkennungsschaltungen für jeweils einen der zwei Schallstrahlen vorgesehen sind, und jede Quadraturerkennungsschaltung (401, 402) enthält:

zwei Multiplizierer (41A, 41B) zum Multiplizieren von Echosignalen, die von Reflexionen an einem Körper oder einer Substanz herrühren, die entlang dem relevanten Schallstrahl angeordnet ist, mit dem jeweiligen Sinus-Signal und dem Cosinus-Signal;

zwei Tiefpassfilter (42A, 42B) zum Eliminieren hoher Frequenzkomponenten, die in den Ausgabesignalen der zwei Multiplizierer (41 A, 41B) enthalten sind, und zum Liefern der tiefen Frequenzkomponenten an die Bereichsgattermittel (401-43A, 43B; 402), welche hohen Frequenzkomponenten bei Reflexionen von allen Körpern oder Substanzen auftreten, die entlang des jeweiligen Schallstrahls angeordnet sind.

zwei Hochpassfilter (44A, 44B) zum Eliminieren von tiefen Frequenzkomponenten von Ausgabesignalen der Bereichsgattermittel(401-43A, 43B; 402), welche tiefen Frequenzkomponenten bei Reflexionen von starren Körpern oder Substanzen auftreten, die entlang des jeweiligen Schallstrahls angeordnet sind;

zwei Quadierschaltungen (45A, 45B) zum Quadrieren von Ausgabesignalen der zwei Hochpassfilter (44A, 44B); und

einer Addierschaltung (46) zum Addieren der Ausgabesignale der zwei Quadrierschaltungen (45A, 45B), um eine Signalspannung zu erzeugen, die zu dem fließenden Blut gehört, daß entlang der relevanten Schallstrahlen angeordnet ist.

3. Korrelationserkennungstyp-Ultraschall-Blutflußmesser nach Anspruch 2, bei dem das Bereichsgatter vier Bereichsgatterschaltungen (401-43A, 43B; 402) umfaßt, zwei der vier Bereichsgatterschaltungen in jede Quadraturerkennungsschaltung der ersten Mittel (401, 402), zwischen den zwei Tiefpassfiltern (42A, 41B) und den zwei Hochpassfiltern (44A, 44B) der betreffenden Quadraturerkennungsschaltung, eingefügt sind,

bei dem jede Bereichsgatterschaltung (43A, 43B) eine Halteschaltung (434, R, C) und eine torgesteuerte Abtastschaltung ( 431, 432, 433 ) umfaßt;

bei dem die Abtastschaltung einen Operationsverstärker (434), einen Widerstand (R), der mit einem Eingabeanschluß des Operationsverstärkers verbunden ist, und einem Kondensator (C) umfaßt, der zwischen dem Eingabeanschluß des Operationsverstärkers und dem Ausgabeanschluß des Operationsverstärkers verbunden ist, welcher Ausgabeanschluß einem Ausgabeanschluß der Bereichsgatterschaltung entspricht;

bei dem die torgesteuerte Abtastschaltung umfaßt :

einen Gatterimpulsgenerator (431) zur Erzeugung eines Gatterimpulses, welcher eine erste Impulsweite hat, die dem ausgedehnten Probevolumen entspricht, jedes mal wenn ein Abtaststeuersignal mit einer zweiten Impulsweite auf den Gatterimpulsgenerator zur Bestimmung der Position des Probevolumens auf dem relevanten Schallstrahl aufgegeben wird, mit einer Wiederholungsfrequenz gleich der der Stöße von Ultraschallwellen, und wenn die zweite Impulsweite beendet ist;

ein erstes Schaltelement (432), um eine Eingabeverbindung, zur Führung von Ausgabesignalen eines Tiefpassfilters (42A, 42B) einer Quadraturerkennungsschaltung zu dem Widerstand (R) der Halteschaltung, EIN zu machen, wenn der Gatterimpuls auf das erste Schaltelement, innerhalb des Zeitraumes der ersten Impulsweite aufgegeben wird; und

ein zweites Schaltelement (433), um eine kurze Verbindung, zur Entladung einer Spannung, die auf dem Kondensator (C) der Halteschaltung geladen war, EIN zu machen, wenn das Abtaststeuersignal auf das zweite Schaltelement innerhalb des Zeitraumes der zweiten Impulsweite aufgegeben wird.

## FIG. 1

## FIG. 6

FIG. 2

## FIG. 3

## FIG. 4

## F/G. 5

# FIG. 7

C₁(t) → G₁(n)

SIN   COS   Vs

41A  42A  43A  44A  45A

41B  42B  43B  44B  45B

46

401

EP 0 177 942 B1

# FIG. 8 (a)

# FIG. 8 (b)